(19) Europäisches Patentamt
European Patent Office
Office européen des brevets

(11) Publication number: **0 017 912**
**B1**

(12) **EUROPEAN PATENT SPECIFICATION**

(45) Date of publication of patent specification: **26.01.83**

(21) Application number: **80101904.3**

(22) Date of filing: **10.04.80**

(51) Int. Cl.[3]: **B 01 J 27/08,**
C 07 D 307/36

(54) **Catalyst for the preparation of furan compounds.**

(30) Priority: **10.04.79 US 29101**
**18.01.80 US 109263**
**18.01.80 US 109264**

(43) Date of publication of application:
**29.10.80 Bulletin 80/22**

(45) Publication of the grant of the patent:
**26.01.83 Bulletin 83/4**

(84) Designated Contracting States:
**BE CH DE FR GB IT LI LU NL**

(56) References cited:
**DE - A - 2 264 462**
**FR - A - 2 250 732**
**FR - A - 2 368 454**
**GB - A - 2 003 878**
**US - A - 4 092 370**

(73) Proprietor: **E.I. DU PONT DE NEMOURS AND COMPANY**
**Legal Department 1007 Market Street**
**Wilmington Delaware 19898 (US)**

(72) Inventor: **Fremont, Joseph Melvin**
**10 Governor Markham Drive R.D. 3**
**Glen Mills, Pennsylvania 19342 (US)**
Inventor: **Garnett, Donald Irwin**
**R.D. 2 Box 46**
**Hockessin, Delaware 19707 (US)**

(74) Representative: **Abitz, Walter, Dr.-Ing. et al,**
**Abitz, Morf, Gritschneder P.O. Box 86 01 09**
**D-8000 München 86 (DE)**

**EP 0 017 912 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Catalysts for the preparation of furan compounds

This invention relates to new catalysts for the preparation of furan compounds, and more particularly for the preparation of furan compounds from diolefins, particularly butadiene.

Furan is a chemical useful in furan resins and, more importantly, as a raw material for the manufacture of tetrahydrofuran. However, furan today is prepared from natural pentose contained in corn or oat hulls through furfural as an intermediate. To reduce the cost of tetrahydrofuran, it is produced today from acetylene and formaldehyde through 1,4-butynediol and 1,4-butanediol as intermediates. While this is a satisfactory process, it is moderately complex in the number of steps required to reach tetrahydrofuran as the final product. More importantly, however, acetylene is becoming more expensive due to energy inefficiences involved in its manufacture.

There have been attempts over the years to produce furan directly by the catalytic oxidation of butadiene. These attempts have generally been under harsh processing conditions, e.g., at temperatures higher than about 375°C, which result in overoxidation to carbon oxides and furan decomposition. Such high temperature, vapor phase processes are exemplified by U.S. Patents 3,238,225; 3,716,545; 3,775,508; 3,864,279; 3,906,009; 3,912,763; 3,928,389 and 4,026,820.

A process for preparing furan by oxidation of butadiene with molecular oxygen at lower temperatures (40—150°C) either in a vapor phase reaction or a liquid phase reaction is described in Japanese Patent Application Publication 52—77049 dated June 29, 197. In one aspect of the process described therein, a palladium salt and a thallium or indium salt are dissolved in acidified water and then butadiene and oxygen are passed through the solution. A similar process is described in Russian Patent 265119 dated June 24, 1970. In this process, butadiene (or butadiene and air) is passed through an acidic, aqueous solution of cupric chloride and palladium chloride at a temperature of 60—110°C. Cuprous chloride can be used in place of palladium chloride. Both of these process suffer deficiencies of impractical rates of reaction and excessively low reaction life.

Dutch Patent Application No. 7710687 describes bimetallic catalysts supported on a solid carrier which are particularly useful for the conversion of olefines to ketones by means of molecular oxygen. The two metallic components of the catalysts may be selected from metal salts or metal complexes. Among many different metal salts or melta complexes there may be used a combination of $PdCl_2$ and $CuCl_2$.

According to the present invention, there is provided a new catalyst comprising a support having deposited thereon

(a) 0.5—30%, by weight of the support, of a mixture of cuprous chloride and cupric chloride,

(b) optionally, 0.5—10%, by weight of the support, of an alkali metal chloride or an alkaline earth metal chloride, and

(c) at least one of

(1) 0.01—2%, by weight of the support of palladium chloride, and

(2) 0.1—2%, by weight of the support, of an iodide compound.

The new catalysts are useful in a process for preparing furan compounds, which process comprises contacting the catalyst in a reaction zone with

(1) a molecular oxygen-containing gas,

(2) a diolefin of the formula

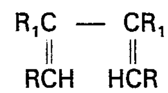

where each R is H or an alkyl group of 1—4 carbon atoms, and

each $R_1$ is H, a halide or an alkyl group of 1—4 carbon atoms,

with the proviso that the total number of carbon atoms does not exceed 8, and

(3) water vapor containing about 0.5—5% by weight of HCl.

Preferably the process comprises: contacting a gaseous stream comprising (1) air, (2) butadiene, and (3) water vapor containing about 0.5—5% by weight HCl based on the weight of the water, in a reaction zone maintained at a temperature in the range of about 80—125°C and at the pressure in the range of about 1—5 atmospheres, with a catalyst according to the invention consisting essentially of a silica, alumina or silica-alumina support having deposited thereon (1) 0.5—30%, by weight of the support, of a mixture of cuprous chloride and cupric chloride (2) 0.1—2%, by weight of the support, of cuprous iodide or 0.01—2%, by weight of the support, of palladium chloride, and (3) 5—7%, by weight of the support, of calcium chloride.

This vapor phase process, which can be conducted at low temperatures and pressures, produces the furan compound at good conversions and yields and at practical rates of reaction.

A 1,3-diolefin is used as the starting material in this process. The useful 1,3-diolefins have the formula

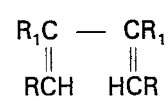

where each R is H or an alkyl group of 1—4 carbon atoms (preferably methyl), and

each $R_1$ is H, an alkyl group of 1—4 carbon atoms (preferably methyl) or a halide such as chloro or iodo (preferably chloro),

with the proviso that the total number of carbon atoms does not exceed 8, preferably does not exceed 5.

Illustrative diolefins are 1,3 - butadiene; 1,3 - pentadiene; 2 - chloro - 1,3 - butadiene; 2 - methyl - 1,3 - butadiene; 2 - iodo - 1,3 - butadiene; 1,3 - hexadiene, 2,4 - hexadiene; 2,3 - dimethyl - 1,3 - butadiene; 3,4 - dimethyl - 2,4 - hexadiene; 4,6 - octadiene; and 1,3 - octadiene. Of these diolefins, the first four listed are preferred due to commercial availability, with 1,3 - butadiene being most preferred. Mixtures of diolefins can be used if desired.

When used in the process, the diolefin can be fed separately and undiluted, mixed with a gas inert to the reaction, such as nitrogen, carbon monoxide or carbon dioxide, or mixed with an oxygen-containing gas such as air. In a preferred embodiment, the diolefin is mixed with the oxygen-containing gas with water vapor containing about 0.5—5% by weight HCl to form a gaseous stream which is then contacted with the catalyst. However, cyclic addition of the gses to a reactor can be employed. While there does not appear to be anything critical about the gaseous stream's compositional make-up, having about 10—50% by volume of water vapor in the gaseous feed has been found useful. The stream contains about 1—50% by volume of butadiene with the balance being air. A preferred HCl content is about 1—2% by weight, based on the weight of water, when palladium is used in the catalyst and about 3—5% by weight, based on the weight of water, when an iodide is used in the catalyst.

The oxygen-containing gas employed can be molecular oxygen as such or molecular oxygen used with a diluent inert to the reaction such as nitrogen or the like. Typical molecular oxygen-containing gases are air, which is preferred, flue gases or synthesis gases which contain residual oxygen, and any source of molecular oxygen free of contaminants detrimental to the desired reaction.

The catalyst of the present invention consists essentially of an inert support, preferably of silica, alumina, silica-alumina or mixtures of these, in the form of pellets, powders or other configurations appropriate for use in a fixed, moving or fluid bed reactor. Illustrative other supports are titania, zirconia and other ceramics well known to those skilled in the art. Deposited on the support is a mixture of cuprous chloride and cupric chloride, optionally an alkali metal chloride or an alkaline earth metal chloride, and at least one of palladium chloride and an iodide compound, typically cuprous iodide. When iodide is deposited on the support, it is usually as cuprous iodide even though iodide is fed to the reaction system as hydrogen iodide or as an alkali metal iodide.

Illustrative of the alkali metal chlorides and the alkaline earth metal chlorides which can be used are sodium chloride, potassium chloride, lithium chloride, calcium chloride, magnesium chloride, strontium chloride and barium chloride. Alkaline earth metal chlorides are preferred for their effectiveness. Calcium chloride is most preferred.

The mixture of cuprous chloride and cupric chloride is present on the support at a concentration in the range of 0.5—30%, preferably 8—12%, by weight of the support. The alkali metal chloride or alkaline earth metal chloride, when it is used, is present at a concentration of 0.5—10%, preferably 5—7%, by weight of the support. Palladium chloride, when it is used, is present at a concentration of 0.01—2%, preferably 0.02—0.06%, by weight of the support. Iodide is present at a concentration of 0.1—2%, by weight of the support.

The catalyst of the invention is conveniently prepared by dissolving cupric chloride and cuprous chloride, and the alkali metal chloride or alkaline earth metal chloride and palladium chloride (when they are used) in a dilute HCl solution. One volume of solution so obtained is added to 2 volumes of catalyst support which absorbs essentially all the added solution. The material is then oven dried, usually under vacuum at 90—100°C. In the event iodide addition is desired, a second impregnation is made using the desired iodide concentration in aqueous solution. A second suitable method involves feeding HI solution in the aqueous HCl feed to the catalyst vapor phase until the desired iodide has been charged.

The process for the preparation of furan compounds can be carried out at a temperature in the range of about 80—125°C, preferably about 100—125°C. As would be expected, rates of furan production are reduced at the lower temperatures. Reaction pressures are typically in the range of about 1—5 atmospheres, preferably about atmospheric pressure. It is the diolefin's partial pressure in the gas stream contacted with the catalyst that determines the particular pressure used.

The diolefin flow rate through the catalyst contained in a reaction vessel does not appear to be critical. As will be apparent, the flow rate should not be so fast as to give inadequate contact time between the diolefin and the catalyst or so slow as to enable the resulting furan product time to decompose or polymerize. It is preferred that the catalyst be in a fixed bed as is known to those skilled in the art, and that the reaction off-gases containing furan product be removed from the reaction vessel promptly. The optimum contact time between the diolefin and catalyst depends on many factors and is readily determined by one skilled in the art.

Since the reaction system is very corrosive,

the reactor for carrying out the process of the invention should be made of a material which is not corroded. Illustrative materials are glass or ceramic-lined metals, titanium, tantalum-clad metals, impregnated graphite tubes and the like.

Another embodiment is a two-step cyclic process wherein furan is produced in a first step by contacting the diolefin with the catalyst, and cuprous ion on the catalyst is oxidized to cupric ion in a second step by contacting the catalyst containing excess cuprous ion with the above-described oxygen-containing gas.

In a representative cyclic operation, a feed of butadiene and nitrogen is contacted with the catalyst until butadiene starts being detected in the off-gases. It is theorized that butadiene is absorbed by the catalyst and takes some intermediate compound form. The butadiene feed is turned off and air is introduced to the reactor and fed in until oxygen consumption drops, indicating that oxidation of the catalyst is essentially complete. The air is then turned off and butadiene again fed to the catalyst. Water with HCl is fed at all times. Such cyclic operation seems to give a higher concentration of furan in the off-gases for a longer period of time. When the furan yield then drops below some predetermined level, air is reintroduced for reoxidation of the catalyst. To maintain a high furan production rate, at least two reactors can be operated sequentially so that while the catalyst in one reactor is being oxidized, furan is being produced in the second reactor. An advantage of cyclic operation is that less palladium can be used in the catalyst. An alternative to cyclic operation is continuous circulation of the catalyst bed.

In the examples, concentrations were determined by gas chromatographic analyses. The samples were injected into a 3.04 m×3.2 mm (10′×1/8″) column of "Poropak* N" (highly crosslinked polystyrene) for determination of their air, carbon dioxide, butadiene and furan contents. Analyses were carried out at 175°C with helium carrer gas at 33 ml/min. The areas of the peaks in the chromatograph were converted to volume percents of components using factors determined by calibrations with known quantities of components. In all of the examples except Example 1, water was removed from the reaction off-gases by an air-cooled condenser prior to gas analysis.

The invention can be further understood by the following examples in which percentages are by volume unless otherwise stated.

Example 1

A solution of 5 g $PdCl_2$, 5 g CuCl, 50 g $CuCl_2 \cdot 2H_2O$ and 15 ml of concentrated HCl in 200 ml water was added to 600 g of 3.2 mm (1/8″) alumina pellets (Houdry 100 S) and oven dried at 100°C for about 12 hours under vacuum. The resulting catalyst was charged to an oil-heated glass reactor.

Gaseous reactants (except water) were mixed in a manifold and fed to the reactor through a glass distributor located in the bottom of the reactor. The water-HCl feed was fed in a tube passing through the top of the reactor and the catalyst bed to preheat the water. At the bottom of the reactor, the water vaporized and mixed with the other gases discharged from the distributor. The feeds to the reactor were (1) butadiene at 10 $cm^3$/min, (2) $N_2$ at 200 $cm^3$/min, (3) air at 125 $cm^3$/min and (4) water vapor with 1% by weight HCl at 250 $cm^3$/min. At a reactor temperature of 95°C, the furan content in the off-gases was 2.8% one hour after feeds were started. The run continued for 17 hours with the furan content in the off-gases ranging from 2.4—4%.

Example 2 (Best mode)

A catalyst was prepared as in Example 1 except the catalyst consisted of 50 g $CuCl_2 \cdot 2H_2O$, 1 g $PdCl_2$, 33 g $CaCl_2$ and 5 g CuCl on 500 g of the alumina pellets.

After the catalyst was charged to the oil-heated glass reactor maintained at a temperature of 95—100°C, the gas feeds were started at the following rates: 12 $cm^3$/min of butadiene, 200 $cm^3$/min of $N_2$, 125 $cm^3$/min of air and 250 $cm^3$/min of water vapor which contained 2% by weight of HCl. For a period of 6 hours, only a trace of furan was present in the off-gases. After this period of time, the butadiene and $N_2$ feeds were turned off and the air feed increased to 250 $cm^3$/min for a period of two hours. After this period, the air feed was turned off and the butadiene feed rate increased to 70 $cm^3$/min. After 20 minutes at the new feed rates, furan was analyzed in the off-gases at a concentration of 7.2%. Furan concentration in the off-gases after 80 minutes was 15% and then declined to 5% after 120 minutes.

Example 3

A catalyst consisting of 100 g $CuCl_2 \cdot 2H_2O$, 10 g CuCl on 400 g of 3,2 mm (1,8″) silica-alumina pellets was charged to the oil-heated reactor described in Example 1.

Temperature was raised to 110°C and 5 ml of 78% HI aqueous solution in 25 ml of 1% aqueous HCl was fed to produce cuprous iodide on the pellets. The catalyst was conditioned at 110°C with a stream of butadiene in nitrogen at 20 $cm^3$ and 200 $cm^3$/min, respectively, for a period of 90 minutes. At that time the butadiene and nitrogen feeds were turned off and the system oxidized with an air flow rate of 250 $cm^3$/min at 110°C for one hour. After that time a feed composition consisting of 200 $cm^3$/min water vapor containing 5% by weight HCl, 70 $cm^3$/min butadiene and 120 $cm^3$/min air was fed at 98°C. After one hour furan was 5% in the product and remained at that level for an additional four hours when the test was terminated.

Example 4

A catalyst, prepared as in Example 1, consisting of 100 g $CuCl_2 \cdot 2H_2O$, 10 g CuCl and 0.1 g $PdCl_2$ on 600 g of 100 S Houdry alumina 3.2 mm (1/8") pellets was charged to the oil-heated glass reactor maintained at 100°C. The feed streams to the reactor were fed as in Example 1 at the following rates: butadiene at 60 $cm^3$/min, $N_2$ at 75 $cm^3$/min and water vapor containing 1% by weight HCl at 300 $cm^3$/min. No air was fed to the reactor. These feeds were continued for 90 minutes with only a trace of furan being detected in the off-gases. At this point, the butadiene and $N_2$ feeds were stopped and the air feed turned on to give a flow rate of 350 $cm^3$/min. The air and water vapor flows continued for 2 hours and then the butadiene and $N_2$ feeds restarted at the above-described rates. At a reactor temperature of 98°C, furan was detected in the off-gases for 2 hours at a concentration ranging from 8—10%. Again, the butadiene and $N_2$ feeds were stopped and the air and water vapor feeds continued for another 1 hour. After the 1 hour, the butadiene and $N_2$ feeds were restarted as above and furan was produced at a concentration of 8% in the off-gases for an additional period of 2 hours.

Example 5

A catalyst consisting of 550 g of steam-treated alumina-silica pellets (sold by Grace Chemicals) impregnated with 100 g $CuCl_2 \cdot 2H_2O$, 20 g CuCl and 0.2 g $PdCl_2$ was prepared as in Example 1. The catalyst was charged to an oil-heated glass reactor as described in Example 1 which was maintained at 117°C.

Water vapor containing 3% by weight HCl was fed to the reactor at a rate of 300 $cm^3$/min. The butadiene and $N_2$ feeds were started at 25 $cm^3$/min and 200 $cm^3$/min, respectively. For a period of 1 hour and 18 minutes, virtually all of the butadiene was absorbed on the catalyst as only traces of furan were detected in off-gases. The butadiene and $N_2$ feeds were turned off and air was fed to the reactor for copper regeneration at a rate of 180 $cm^3$/min. During 111 minutes of feeding air, only a trace of furan was produced. The air feed was then turned off and the butadiene and $N_2$ feeds restarted at 70 $cm^3$/min and 75 $cm^3$/min, respectively. After 34 minutes, the furan concentration in the off-gases rose to a maximum of 12.6% and then dropped to 4% after 84 minutes. At this point, the $N_2$ feed was turned off and air feed was restarted at a rate of 180 $cm^3$/min. Furan concentration in the off-gases increased to 5.7% and remained constant during the next 3-hour period before the run was terminated.

## Claims

1. A catalyst comprising a support having deposited thereon
(a) 0.5—30%, by weight of the support, of a mixture of cuprous chloride and cupric chloride,
(b) optionally, 0.5—10%, by weight of the support, of an alkali metal chloride or an alkaline earth metal chloride, and
(c) at least one of
  (1) 0.01—2%, by weight of the support of palladium chloride, and
  (2) 0.1—2%, by weight of the support, of an iodide compound.

2. The catalyst of claim 1 in which the component in (c) is palladium chloride.

3. The catalyst of claim 1 in which the component in (c) is cuprous iodide.

4. The catalyst of claim 1 in which the alkaline earth metal chloride is calcium chloride.

5. A catalyst comprising a support having deposited thereon
(a) 0.5—30%, by weight of the support, of a mixture of cuprous chloride and cupric chloride,
(b) 0.5—10%, by weight of the support, of calcium chloride, and
(c) 0.01—2%, by weight of the support, of palladium chloride.

## Patentansprüche

1. Katalysator, der einen Träger enthält, auf dem abgelagert sind:
(a) 0,5—30% des Gewichts des Trägers eines Gemischs von Kupfer-I-chlorid und Kupfer-II-chlorid,
(b) gegebenenfalls 0,5—10% des Gewichts des Trägers eines Alkalimetallchlorids oder eines Erdalkalimetallchlorids und
(c) mindestens eines von
  (1) 0,01—2% des Gewichts des Trägers von Palladiumchlorid und
  (2) 0,1—2% des Gewichts des Trägers einer Jodidverbindung.

2. Katalysator nach Anspruch 1, in dem die Komponente (c) Palladiumchlorid ist.

3. Katalysator nach Anspruch 1, in dem die Komponente (c) Kupfer-I-jodid ist.

4. Katalysator nach Anspruch 1, in dem das Erdalkalimetallchlorid Calciumchlorid ist.

5. Katalysator, der einen Träger enthält, auf dem abgelagert sind
(a) 0,5—30% des Gewichts des Trägers eines Gemisches von Kupfer-I-chlorid und Kupfer-II-chlorid,
(b) 0,5—10% des Gewichts des Trägers an Calciumchlorid und
(c) 0,01—2% des Gewichts des Trägers an Palladiumchlorid.

## Revendications

1. Un catalyseur comprenant un support sur lequel sont déposés:
(a) de 0,5 à 30% en poids, par rapport au support, d'un mélange de chlorure cuivreux et chlorure cuivrique.
(b) facultativement, de 0,5 à 10% en poids, par

rapport au support, d'un chlorure de métal alcalin ou d'un chlorure de métal alcalino-terreux, et

(c) au moins l'un des composants suivants

(1) de 0,01 à 2% en poids, par rapport au support, de chlorure de palladium, et

(2) de 0,01 à 2% en poids, par rapport au support, d'un iodure.

2. Le catalyseur selon la revendication 1 dans lequel le composant en (c) est le chlorure de palladium.

3. Le catalyseur selon la revendication 1 dans lequel le composant en (c) est l'iodure cuivreux.

4. Le catalyseur selon la revendication 1 dans lequel le chlorure de métal alcalino-terreux est le chlorure de calcium.

5. Un catalyseur comprenant un support sur lequel sont déposés:

(a) de 0,5 à 30% en poids, par rapport au support d'un mélange de chlorure cuivreux et chlorure cuivrique,

(b) de 0,5 à 10% en poids, par rapport au support, de chlorure de calcium, et

(c) de 0,01 à 2% en poids, par rapport au support, de chlorure de palladium.